# EUROPEAN PATENT SPECIFICATION

(11) **EP 0 497 609 B1**
(45) Date of publication and mention of the grant of the patent: **12.04.1995**
(21) Application number: 92300817.1
(22) Date of filing: 30.01.1992
(51) Int. Cl.: A61K 31/54

(54) **Pyrimido-benzothiazine derivatives for the treatment of inflammation, allergy and cardiovascular disease**
Pyrimido-benzothiazinderivate zur Behandlung von Entzündung, Allergie and kardiovaskulären Erkrankung
Dérivés de pyrimido-benzothiazine pour le traitment de l'inflammation, de l'allergie et la maladie cardiovasculaire

(30) Priority: 01.02.1991 JP 12268/91
(43) Date of publication of application: 05.08.1992
(73) Proprietor: PFIZER INC., New York, N.Y. 10017 (US)
(72) Inventor: Hayakawa, Takako, Chita-City, Aichi 478 (JP); Shishido, Yuji, Chita-gun, Aichi 470-32 (JP); Sakakibara, Minoru, Chita-gun, Aichi 470-23 (JP); Shimada, Kaoru, Tokyo 156 (JP)
(74) Representative: Wood, David John

(56) References cited:
- EP-A- 0 140 709
- WO-A-88/04658
- J. CHEM. SOC., CHEM. COMMUN., vol. 16, 1977, pages 557-558; T. HIRAMITSU et al.: "Synthesis of 10-thiaisoalloxazines"
- CHEM. PHARM. BULL., vol. 32, no. 6, 1984, pages 2474-2476; M. SAKO et al.: "Aversatile and convenient method for the synthesis of pyrimido[4,5-b][1,4]thiazines"
- CHEM. PHARM. BULL., vol. 34, no. 2, 1986, pages 664-668; M. SAKO et al.:"Reactions of 10-thiaisoalloxazines with alkyl amines and benzyl alcohol"
- CHEM. PHARM. BULL., vol. 37, no. 8, 1989, pages 2197-2199; T. ITOH et al.:"Synthesis of some tricyclic heterocycles from 5,6-diamino-1,3-dimethyluracil"

## Description

This invention relates to new uses for certain pyrimido-benzothiazine derivatives and the pharmaceutically-acceptable salts thereof in the treatment or alleviation of inflammatory diseases, allergy and cardiovascular diseases in a mammal. This invention also relates to pharmaceutical compositions comprising certain pyrimido-benzothiazine derivatives or a pharmaceutically-acceptable salt thereof which compositions are useful in the treatment or alleviation of inflammatory diseases, allergy and cardiovascular diseases in a mammal.

Arachidonic acid is known to be the biological precursor of several groups of endogenous metabolites, prostaglandins including prostacyclins, thromboxanes and leukotrienes. The first step of arachidonic acid metabolism is the release of arachidonic acid and related unsaturated fatty acids from membrane phospholipids via the action of phospholipase A₂.

The metabolic pathways of the thus released arachidonic acid consist of the one through which arachidonic acid is converted into endoperoxide (PGG₂) by cyclooxygenase and the one through which arachidonic acid is converted into 5-hydroperoxyeicosatetraenoic acid (5-HPETE) by lipoxygenase. PGG₂ is further converted into prostacyclin (PGI₂), PGF₂, PGD₂, TXA₂ and TXB₂ through three different metabolic pathways. On the other hand, 5-HPETE is converted into leukotrienes.

The substances produced through these metabolic pathways have various physiological effects. For example, prostaglandins (PGs) exhibit a wide variety of physiological effects such as renin secretion stimulation, FFA release regulation, vasoconstriction, vasodilation, nervous conduction, and others. It is thought that PGs often exhibit these effects by participating in the increase or decrease of the intracellular concentration of cyclic AMP. It is known that thromboxanes, which are synthesized in platelets, cause vasoconstriction and platelet agglutination. Furthermore, it is thought that leukotrienes serve as a medium of allergic reaction and inflammation, and that they are released when a specific allergen binds to immunoglobulin E (IgE) antibody on the surfaces of mast cells for example. It is also known that leukotrienes cause bronchial constriction, arteriolar constriction and accelerated vascular permeability, and draw neutrophils and eosinophils up to the site of inflammation.

These pathological physiological changes in which leukotrienes participate are observed in patients with inflammatory diseases including chronic rheumatoid arthritis, gout, ischemia reperfusion injury, psoriasis and inflammatory bowel disease. For example, in the articular fluids of patients with rheumatoid arthritis, leukotrienes of higher levels than the normal level are detected and immune complexes consisting of IgG and rheumatoid factors, and multinuclear spheres having taken in large amounts of the immune complexes are observed.

Medication, plasma replacement, physiotherapy and surgical therapy are known methods for treating rheumatoid arthritis and adrenocorticosteroids and nonsteroidal anti-inflammatory agents such as indomethacin are preferred drugs for medication because of their rapid action.

However, steroids should not be used as far as possible because of their side effects and the difficulty of a breakaway from them and when using a steroid it is considered better to use it for a very short period or to locally inject it. This is so because a steroidal anti-inflammatory agent such as dexamethasone binds to receptor protein in the cytoplasm and inhibits the effect of phospholipase A₂. Inhibition of phospholipase A₂ results in the release of arachidonic acid from membrane phospholipids being inhibited and thereby results in inhibition of the production of the cyclooxgenase products (PGs and TXs) and the lipoxygenase products (LTs), the metabolic products of arachidonic acid. On the other hand, nonsteroidal anti-inflammatory agents, which inhibit the production of endoperoxide from arachidonic acid by inhibiting the effect of cyclooxygenase, consequently inhibit the production of prostaglandins and thromboxanes similar to steroidal anti-inflammatory agents.

In man, it is believed that inhibition of the metabolism of arachidonic acid makes unstable the homeostasis of the internal environment achieved by prostaglandins, possibly contributing to the side effects caused by administration of adrenocortiocosteroids or nonsteroidal anti-inflammatory agents.

Because of the above reasons, it has conventionally been desired to develop compounds which specifically inhibit the production of leukotrienes without inhibiting the production of prostaglandins and thromboxanes.

Recently several review articles on lipoxygenase inhibitors have been reported. See H. Masamune and L. S. Melvin, Sr., in: Annual Reports in Medicinal Chemistry 24 (1989) pp. 71-80 (Academic); and B. J. Fitzsimmons and J. Rokach in: Leukotrienes and Lipoxygenases (1989) pp. 427-502 (Elsevier).

However, it is believed that the clinical range of applications for said lipoxygenase inhibitors is limited. It is therefore desirable to develop novel lipoxygenase inhibitors.

Pyrimido-benzothiazine derivative compounds of the formulae 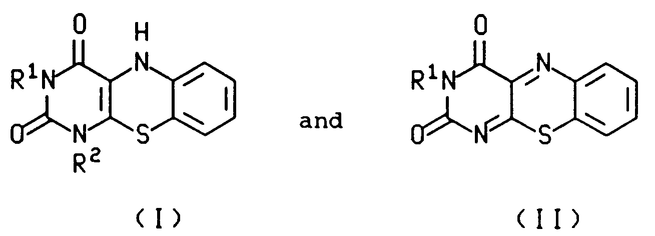
wherein R¹ and R² are each, independently, hydrogen or methyl are known as analogs of the main chemical components in flavin, a significant coenzyme in a living body. A synthetic method for the preparation of said pyrimido-benzothiazine derivative compounds has been established and used. However, until the invention hereof, it was not known that such pyrimido-benzothiazine derivative compounds inhibit the action of lipoxygenase and are useful in the treatment or alleviation of inflammatory diseases, allergy and cardiovascular diseases in a mammal.

The present invention relates to new uses of compounds of the formulae 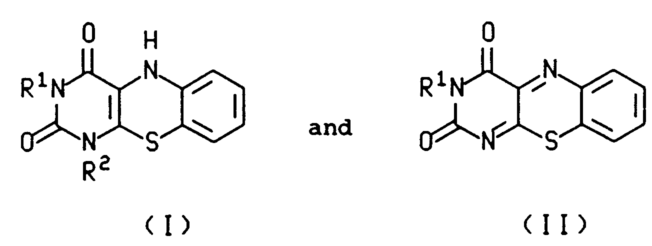
and the pharmaceutically-acceptable salts thereof wherein R¹ and R² are each, independently, hydrogen or methyl for the treatment or alleviation of inflammatory diseases, allergy and cardiovascular diseases in a mammal. Preferred compounds are those of formula (I) wherein R¹ and R² are each hydrogen; R¹ and R² are each methyl or R¹ is methyl and R² is hydrogen. Also preferred are compounds of formula (II) wherein R¹ is hydrogen or methyl. Particularly preferred is the compound of formula (II) wherein R¹ is methyl.

The present invention also relates to pharmaceutical compositions for the treatment or alleviation of inflammatory diseases, allergy or cardiovascular diseases in a mammal which compositions comprise an effective amount of a compound of formulae (I) or (II), as defined above, or a pharmaceutically-acceptable salt thereof and a pharmaceutically-acceptable diluent or carrier.

The term "pharmaceutically-acceptable salt" as used herein means a non-toxic cation salt including those of alkaline earth metals such as sodium, lithium, calcium and magnesium, and organic cation bases of ammoniums and amines, or non-toxic acid salts, for example, the hydrochloride, hydrobromide, sulfate or bisulfate, phosphate or acid phosphate, acetate, citrate, fumarate, gluconate, lactate, maleate, succinate, tartrate, methanesulfonate, benzenesulfonate, toluenesulfonate, and formate salts thereof.

The compounds of formulae (I) and (II), above, can be prepared by any of a number of synthetic methods. A preferred method is shown in Scheme A and described below wherein R¹ and R² are as described above. 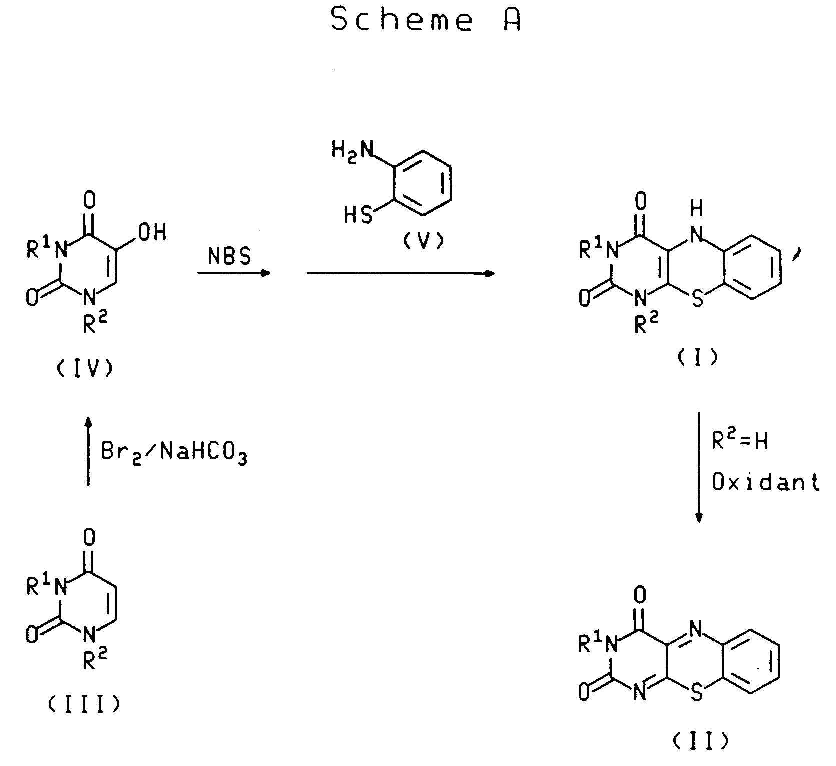
According to the synthetic method shown in Scheme A, above, bromine is added dropwise to an ice-cooled solution or suspension of a pyrimidine-2,4(1H,3H)-dione derivative of formula (III) in water until the solution or suspension is colored pale yellow. The pyrimidine-2,4(1H,3H)-dione derivatives of formula (III) are prepared, for example, by the methods described in Heterocyclic Compounds 16, The pyrimidine supplement II, D. J. Brown, An Interscience Publication, John Willy & Sons, New York, 1985, Table LVIII, page 728. To the resulting pale yellow solution or suspension is carefully added an ice cold saturated aqueous NaHCO₃ solution. The resulting reaction mixture is refluxed, the solvent is removed and the residue is recrystallized from an appropriate solvent or chromatographed over silica-gel to give a 5-hydroxypyrimidine-2,4(1H,3H)-dione derivative of formula (IV).

Then, to a solution or suspension of a 5-hydroxypyrimidine-2,4(1H,3H)-dione derivative of formula (IV) in a reaction inert solvent is added N-bromosuccinimide portionwise at room temperature. A preferred solvent is ethanol. The reaction mixture is stirred until the derivative of formula (IV) disappears. Then, a 2-aminobenzenethiol derivative of formula (V) is added and the mixture is heated at reflux. Certain 2-aminobenzethiol derivatives of formula (V) are commercially available and, further, the derivatives of formula (V) can be prepared from the corresponding 2-aminobenzothiazoles by hydrolysis as described by Mital, R. L., et al., J. Chem. Soc. (c), 2148 (1969). When a precipitate results after reflux of the reaction mixture, the precipitate is collected to give a 1,5-dihydro-2H-pyrimido[4,5-b][1,4]benzothiazine-2,4(3H)-dione derivative of formula (I). When no precipitate is obtained after reflux, the derivative of formula (I) is obtained by chromatographic separation on silica gel. The foregoing method for reacting derivatives of formula (IV) with derivatives of formula (V) to yield derivatives of formula (I) is analogous to the method described by Sako, M., et al., Chem. Pharm. Bull., 32, 2474 (1984).

When derivative compounds of formula (II) are desired, a solution or suspension of a 1,5-dihydro-2H-pyrimido[4,5-b][1,4]benzothiazine-2,4(3H)-dione derivative of formula (I) wherein R² is hydrogen in acetonitrile is prepared. To that solution or suspension at room temperature is added, dropwise, an oxidant such as diethylazodicarboxylate, 1,4-benzoquinone or 2,3-dichloro-5,6-dicyano-1,4-benzoquinone. Alternatively, oxygen can be used as the oxidant. A preferred oxidant is diethylazodicarboxylate. The reaction mixture is stirred. When a precipitate results, the precipitate is collected and washed with diethylether to give a 2H-pyrimido[4,5-b][1,4]benzothiazine-2,4(3H)-dione derivative of formula (II). When no precipitate is obtained after oxidation, the solvent is removed from the reaction mixture, and the residue is triturated with diethylether and filtered to give a compound of formula (II).

The pharmaceutically-acceptable salts of the compounds of the present invention are readily prepared by contacting said compounds with a stoichiometric amount of, in the case of non-toxic cation, an appropriate metal hydroxide or alkoxide or amine in either an aqueous solution or a suitable organic solvent; or, in the case of a non-toxic acid salt, an appropriate mineral or organic acid in either an aqueous solution or a suitable organic solvent. The respective salt may then be obtained by precipitation or by evaporation of the solvent.

The compounds of formulae (I) and (II) of this invention can be shown to inhibit lipoxygenase according to various testing methods. Such methods include the following:
RPC (Rat Peritoneal Cavity resident cells) assay according to methods described in Jap. J. Inflammation 7:145-150 (1987), "Synthesis of leukotrienes by peritoneal macrophages";
PAF (Platelet Activating Factor) lethality assay according to methods described in Prostaglandisis 30(4):545-551 (1985); and
y-RFE (yeast induced Rat Foot Edema) assay according to methods described in Biochem. Pharmacol. 36:547 (1987).

As a result of the RPC assay, certain preferred compounds gave low IC₅₀ values, in the range of 1.4 to 5.3 »M as shown in the Table below. The term "IC₅₀" herein means 50% inhibitory concentration to lipoxygenase activity of the compound tested.

**TABLE**

| Compound of Example No. | RPC IC₅₀ (»M) | PAF lethality survival (%) Dose 50 mg/kg | y-RFE edema inhibitory (%) Dose 60 mg/kg |
|---|---|---|---|
| 1 | 5.3 | 56 | -- |
| 2 | 1.9 | 30 | -- |
| 5 | 1.4 | 69 | 69 |

In the PAF lethality assay, the compound of Example 5, as shown in the Table above, gave a high (69%) survival percentage. The term "survival percentage" herein means the inhibitory efficacy to shock death of the mouse through lipoxygenase metabolites activated by dosing with PAF.

Furthermore, in the y-RFE assay, the compound of Example 5, as shown in the Table above, gave a high (69%) edema inhibitory percentage. The term "edema inhibitory percentage" herein means the inhibitory efficacy to the swelling of inflammatory edema of the rat foot induced by the injection of yeast suspension.

Acute toxicity of the compound of Example 5 was investigated 7 hours after oral administration at 600 mg/kg to male ICR mice (Charles River Japan Inc., 5 weeks old, avg. body weight of 28 g). Consequently, a dosing level of 600 mg/kg, a level ten-fold above the ED₅₀ value in the PAF lethality assay (51 mg/kg), did not show any acute toxic symptoms.

The ability of the compounds of the present invention to inhibit the lipoxygenase enzyme makes them useful for controlling the symptoms induced by the endogenous metabolites arising from arachidonic acid in a mammalian subject. The compounds, therefore, are valuable in the prevention and treatment of such disease states in which the accumulation of arachidonic acid metabolites are the causative factor, e.g., allergic bronchial asthma, skin disorders, rheumatoid arthritis, osteoarthritis and thrombosis.

Thus, the compounds of the present invention, and their pharmaceutically-acceptable salts are of particular use in the treatment or alleviation of inflammatory diseases, allergy and cardiovascular diseases in a human subject as well as in the inhibition of the lipoxygenase enzyme.

For treatment of the various conditions described above, the compounds of this invention and their pharmaceutically-acceptable salts can be administered to a human subject either alone, or, preferably, in combination with pharmaceutically-acceptable carriers or diluents in a pharmaceutical composition according to standard pharmaceutical practice. A compound of this invention can be administered by a variety of conventional routes of administration including oral and parenteral administration and by inhalation. When the compounds are administered orally, the dose range will be from about 0.1 to 20 mg/kg body weight of the subject to be treated per day, preferably from about 0.1 to 5.0 mg/kg per day in single or divided doses. If parenteral administration is desired, then an effective dose will be from 0.1 to 1.0 mg/kg body weight of the subject to be treated per day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of the invention and their pharmaceutically-acceptable salts can be administered, for example, in the form of tablets, powders, lozenges, syrups or capsules, or as an aqueous solution or suspension. In the case of tablets for oral use, carriers which are commonly used include lactose and corn starch. Further, lubricating agents, such as magnesium stearate, are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added. For intramuscular, intraperitoneal, subcutaneous or intravenous use, a sterile solution of the active ingredient is prepared, and the pH of the solutions should be suitably adjusted and buffered. For intravenous use, the total concentration of solutes should be controlled to make the preparation isotonic.

The present invention is illustrated by the following examples. However, it should be understood that the invention is not limited to the specific details of these examples. Proton nuclear magnetic resonance spectra (NMR) were measured at 270 MHz unless otherwise indicated and peak positions are expressed in parts per million (ppm) downfield from tetramethylsilane. The peak shapes are denoted as follows: s, singlet; d, doublet; t, triplet; q, quartet; m, multiplet; br, broad.

### EXAMPLE 1

### 1,5-Dihydro-2H-pyrimido[4,5-b][1,4]benzothiazine-2,4(3H)-dione

To a solution of commercially available 5-hydroxypyrimidine-2,4(1H,3H)-dione (1.025 g) in 60 ml ethanol was added 1.566 g of N-bromosuccinimide in portions at room temperature. The reaction mixture was stirred for 30 minutes. Then, 1.502 g of commercially available 2-aminobenzenethiol was added to the reaction and the mixture was heated at reflux for one hour. After cooling, the precipitate was collected to give 1,5-dihydro-2H-pyrimido(4,5-b)(1,4)benzothiazine-2,4(3H)-dione (1.44 g, 77% yield).
m.p.: >300°C
NMR: (DMSO-d₆) δ: 11.28 (1H, s), 11.02 (1H, s), 7.52 (1H, s), 6.91-6.98 (3H, m), 6.75 (1H, m), 3.29 (3H, s).

### EXAMPLE 2

### 1,5-Dihydro-3-methyl-2H-pyrimido[4,5-b][1,4]benzothiazine-2,4(3H)-dione

Employing the procedure of Example 1 with 5-hydroxy-3-methylpyrimidine-2,4(1H,3H)-dione and 2-amino-benzenethiol yielded the title compound.
m.p.: >300°C (dec.)
NMR: (DMSO-d₆) δ: 11.35 (1H, s), 7.56 (1H, s), 6.92-7.00 (3H, m), 6.74 (1H, m), 3.14 (3H, s).

### EXAMPLE 3

### 1,5-Dihydro-1,3-dimethyl-2H-pyrimido[4,5-b][1,4]benzothiazine-2,4(3H)-dione

Employing the procedure of Example 1 with 5-hydroxy-1,3-dimethylpyrimidine-2,4(1H,3H)-dione and 2-aminobenzenethiol yielded the title compound.
m.p.: 227°C (dec.)

### EXAMPLE 4

### 2H-Pyrimido[4,5-b][1,4]benzothiazine-2,4(3H)-dione

A solution of 2.30 g of 1,5-dihydro-2H-pyrimido[4,5-b][1,4]benzothiazine-2,4(3H)-dione, prepared as described in Example 1, in 250 ml of acetonitrile was oxidized by diethylazodicarboxylate to yield the title compound (1.961 g, 86% yield).
m.p.: 209°C (dec.)
NMR: (DMSO-d₆) δ: 11.72 (1H, s), 8.13 (1H, dd, 8Hz, 1.5Hz), 7.97 (1H, dd, 8Hz, 1.5Hz), 7.81 (1H, dt, 1.5Hz, 8Hz), 7.73 (1H, dt, 1.5Hz, 8Hz), 3.33 (3H, s).

### EXAMPLE 5

### 3-Methyl-2H-pyrimido[4,5-b][1,4]benzothiazine-2,4(3H)-dione

Employing the procedure of Example 4 with the compound produced according to Example 2 yielded the title compound.
m.p.: 270°C (dec.)
NMR: (DMSO-d₆) δ: 8.17 (1H, d, 8Hz), 8.00 (1H, dd, 1.4Hz, 8Hz), 7.73-7.85 (2H, m), 3.26 (3H, s).

## Claims

1. The use of a compound of the formula (I) or (II): 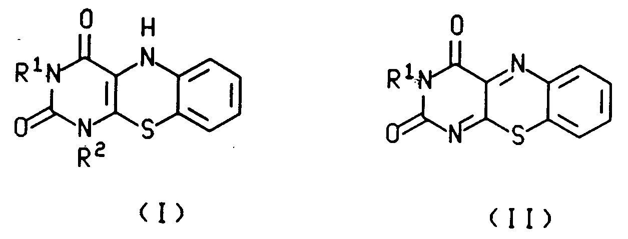 or of a pharmaceutically-acceptable salt thereof, wherein R¹ and R² are each independently hydrogen or methyl, for the manufacture of a medicament for treating inflammatory disease, allergy, or cardiovascular disease.

2. The use according to claim 1 in which compound (I) wherein R¹ and R² are each hydrogen or R¹ and R² are each methyl is used.

3. The use according to claim 1 in which compound (I) wherein R¹ is methyl and R² is hydrogen is used.

4. The use according to claim 1 in which compound (II) wherein R¹ is hydrogen is used.

5. The use according to claim 1 in which compound (II) wherein R¹ is methyl is used.

## Patentansprüche

1. Verwendung einer Verbindung der Formel (I) oder (II): 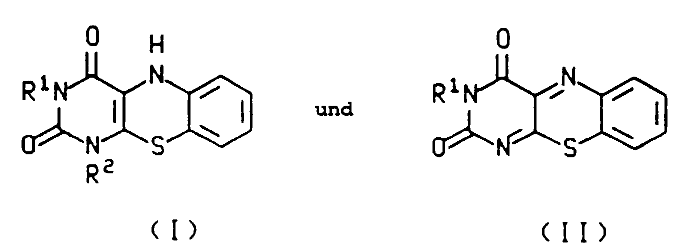 oder eines pharmazeutisch verträglichen Salzes davon, worin R¹ und R² jeweils unabhängig Wasserstoff oder Methyl bedeuten, zur Herstellung eines Medikaments zur Behandlung von entzündlicher Erkrankung, Allergie oder kardiovaskulärer Erkrankung.

2. Verwendung nach Anspruch 1, wobei Verbindung (I), worin R¹ und R² jeweils Wasserstoff darstellen oder R¹ und R² jeweils Methyl bedeuten, verwendet wird.

3. Verwendung nach Anspruch 1, wobei Verbindung (I), worin R¹ Methyl bedeutet und R² Wasserstoff darstellt, verwendet wird.

4. Verwendung nach Anspruch 1, wobei Verbindung (II), worin R¹ Wasserstoff bedeutet, verwendet wird.

5. Verwendung nach Anspruch 1, wobei Verbindung (II), worin R¹ Methyl bedeutet, verwendet wird.

## Revendications

1. Utilisation d'un composé de formule (I) ou (II) : 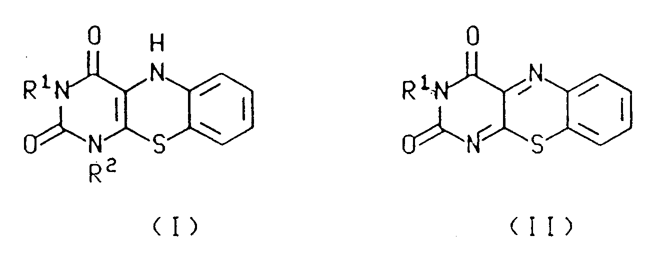 ou d'un de ses sels pharmaceutiquement acceptables, formules dans lesquelles R¹ et R² représentent chacun indépendamment l'hydrogène ou un groupe méthyle, pour la production d'un médicament destiné au traitement d'une maladie inflammatoire, de l'allergie ou d'une maladie cardiovasculaire.

2. Utilisation suivant la revendication 1, dans laquelle est utilisé un composé (I) dans lequel R¹ et R² représentent chacun l'hydrogène ou bien R¹ et R² représentent chacun un groupe méthyle.

3. Utilisation suivant la revendication 1, dans laquelle est utilisé un composé (I) dans lequel R¹ représente un groupe méthyle et R² représente l'hydrogène.

4. Utilisation suivant la revendication 1, dans laquelle est utilisé un composé (II) dans lequel R¹ représente l'hydrogène.

5. Utilisation suivant la revendication 1, dans laquelle est utilisé un composé (II) dans lequel R¹ représente un groupe méthyle.
